(19) Europäisches Patentamt
European Patent Office
Office européen des brevets

(11) EP 0 562 945 B1

(12) **FASCICULE DE BREVET EUROPEEN**

(45) Date de publication et mention de la délivrance du brevet: **07.02.1996 Bulletin 1996/06**

(51) Int. Cl.$^6$: **A61K 7/00**

(21) Numéro de dépôt: **93400742.8**

(22) Date de dépôt: **23.03.1993**

(54) **Utilisation en cosmétique, en particulier dans le maquillage du 5H-dinaphto[2,3-a:2',3'-c]carbazole-6,11,12,17-tétrone**

Verwendung von 5H-Dinaphto[2,3-a:2',3'-c]Carbazole-6,11,12,17-Tetrone in Kosmetika, insbesondere in Schminke

Use of 5H-Dinaphto[2,3-a:2',3'-c]carbazole-6,11,12,17-tetrone in cosmetic, in particular in make-up composition

(84) Etats contractants désignés:
**AT BE CH DE DK ES FR GB GR IE IT LI NL PT SE**

(30) Priorité: **25.03.1992 FR 9203613**

(43) Date de publication de la demande:
**29.09.1993 Bulletin 1993/39**

(73) Titulaire: **L'OREAL**
**F-75008 Paris (FR)**

(72) Inventeurs:
- **Philippe, Michel**
  **F-92160 Antony (FR)**
- **Hocquaux, Michel**
  **F-75012 Paris (FR)**

(74) Mandataire: **Casalonga, Axel et al**
**D-80469 München (DE)**

(56) Documents cités:
- **STN SERVEUR DE BASES DE DONNEES, Karlsruhe DE, FICHIER CHEMICAL ABSTRACTS, vol. 111, no. 25, résumé no. 232499h, Columbus, Ohio, US; M. GRAZIA CORRADINI et al. : "Acid-catalyzed reactions of indoles with 1,4-quinones", & GAZZ. CHIM. ITAL., 1989, 119(3), 153-6**
- **STN SERVEUR DE BASES DE DONNEES, Karlsruhe DE, FICHIER CHEMICAL ABSTRACTS, vol. 81, no. 13, résumé no. 77768m, Columbus, Ohio, US; K.K. PRASAD: "Reinvestigation of the reaction of indole with 1,4-quinones", & TETRAHEDRON LETT.,1974, (15), 1361-2 (Cat. D)**

EP 0 562 945 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet l'utilisation en cosmétique du 5H-dinaphto[2,3-a:2',3'-c]carbazole-6,11,12,17-tétrone, aux compositions cosmétiques le contenant et au procédé de traitement cosmétique le mettant en oeuvre.

On est à la recherche dans le domaine cosmétique et en particulier pour les compositions de maquillage telles des compositions de fond de teint, des crèmes teintées, des mascaras, des fards à joues et à paupières, des rouges à lèvres, des vernis à ongles, de pigments susceptibles de donner à ces différents types de produits une coloration reproductible dans le temps, ayant une granulométrie fine, insolubles dans la plupart des milieux cosmétiques utilisés tels que l'eau et les solvants cosmétiquement acceptables. Ces pigments doivent par ailleurs être stables à des pH compris entre 5 et 9 habituellement utilisés ou rencontrés dans le domaine cosmétique.

La demanderesse vient de découvrir que l'utilisation à titre de pigment du 5H-dinaphto[2,3-a:2',3'-c]carbazole-6,11,12,17-tétrone résultant de la condensation de 2 moles de la 1,4-naphtoquinone avec l'indole est particulièrement intéressante dans le domaine cosmétique et en particulier dans son utilisation dans les compositions de cosmétologie notamment les fonds de teint, crèmes teintées, mascaras, fards à joues et à paupières, rouges à lèvres et vernis à ongles. Ce pigment de structure bien définie permet d'obtenir une' couleur rouge, recherchée dans le domaine cosmétique, bien reproductible, liée à la bonne définition du composé.

Elle a constaté que l'utilisation de ce pigment dans les compositions cosmétiques permettait de leur conférer une couleur rouge intéressante et particulièrement stable notamment à la lumière dans les conditions habituelles de stockage et d'utilisation de ces compositions.

La préparation de ce pigment est plus particulièrement décrite dans K.K. Prasad Tetrahedron letters No. 15, pages 1361-1362, 1974.

Un objet de l'invention est constitué par l'utilisation de ce pigment dans des compositions cosmétiques.

L'invention a également pour objet les compositions cosmétiques contenant ce pigment, ainsi que leur application pour le traitement et la coloration des matières kératiniques telles que la peau ou les ongles.

D'autres objets de l'invention apparaîtront à la lecture de la description et des exemples qui suivent.

Le pigment utilisé conformément à l'invention dans des compositions cosmétiques destinées au traitement de la peau et/ou de ongles est caractérisé par le fait qu'il s'agit du 5H-dinaphto[2,3-a:2',3'-c] carbazole-6,11,12,17-tétrone répondant à la formule :

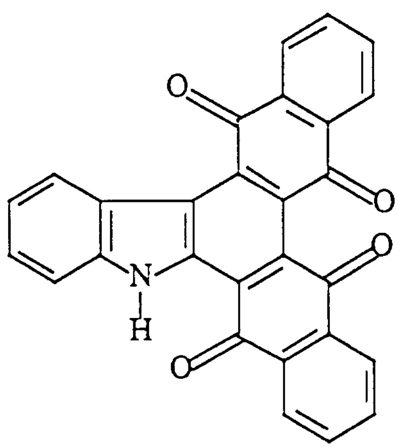

(I)

Ce pigment est utilisé plus particulièrement dans les compositions de fond de teint, des crèmes teintées, des mascaras, des fards à joues et à paupières, des rouges à lèvres, des vernis à ongles. Il peut être utilisé seul ou en mélange avec d'autres pigments minéraux ou organiques classiques.

Les compositions cosmétiques en particulier les maquillages conformes à l'invention sont caractérisées par le fait qu'elles contiennent au moins du 5H-dinaphto[2,3-a:2',3'-c]carbazole-6,11,12,17-tétrone répondant à la formule (I) définie ci-dessus dans un milieu cosmétiquement acceptable.

Le pigment de formule (I) est utilisé généralement à une concentration pouvant varier entre 0,1 et 80% et de préférence entre 1 et 30% en poids par rapport au poids total de la composition.

Le milieu cosmétiquement acceptable est un milieu essentiellement non solvant du pigment.

On appelle milieu essentiellement non solvant un milieu qui dissout moins de 1% dudit pigment.

Les compositions peuvent se présenter sous forme de lotion, de lotion épaissie, de gel, de crème, de poudre, de lait, de stick. Elles peuvent éventuellement être conditionnées en aérosol et se présenter sous forme de mousse ou de spray.

Des compositions sont de préférence des émulsions de type huile-dans-eau ou eau-dans-huile ou des dispersions liposomales ou encore des préparations solides.

Lorsqu'elles sont utilisées sous forme d'émulsions, elles peuvent contenir en outre des agents tensio-actifs bien connus dans l'état de la technique tels que des agents tensio-actifs anioniques, non ioniques, cationiques ou amphotères ou leurs mélanges.

Ces compositions peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anionique, cationiques, anioniques, amphotères ou leurs mélanges, des propulseurs, des agents alcalinisants ou acidifiants.

Les corps gras peuvent être constitués par une huile ou une cire ou leurs mélanges, les acides gras, les alcools gras, la vaseline, la paraffine, la lanoline, la lanoline hydrogénée, la lanoline acétylée.

Les huiles sont choisies parmi les huiles animales, végétales, minérales, de synthèse et notamment l'huile de palme hydrogénée, l'huile de riçin hydrogénée, l'huile de vaseline, l'huile de paraffine, l'huile de purcellin.

Les cires sont choisies parmi les cires animales, fossiles, végétales, minérales ou de synthèse. On peut citer plus particulièrement, les cires d'abeilles, les cires de carnauba, de candelilla, de canne à sucre, du Japon, les ozokérites, la cire de montan, les cires microcristallines, les paraffines.

Lorsque les compositions sont utilisées pour la coloration des ongles, elles se présentent sous forme de produits dits "vernis à ongles" contenant le pigment conforme à l'invention sous forme dispersée dans un solvant cosmétiquement acceptable contenant une ou plusieurs résines et des ingrédients habituellement utilisés dans ce type de produits.

Les compositions conformes à l'invention peuvent également contenir en plus du pigment défini ci-dessus, d'autres pigments généralement utilisés en cosmétique notamment des pigments blancs ou colorés, des pigments nacrés et/ou nacrants permettant de faire varier les colorations ou encore d'augmenter la protection vis-à-vis du rayonnement ultraviolet. Parmi ces pigments on peut utiliser des nanopigments d'oxydes métalliques, tels que les oxydes de titane, de zinc, de cérium et/ou de zirconium ayant un diamètre moyen inférieur à 100 nm de préférence compris entre 5 et 50 nm.

Un autre objet de l'invention est constitué par un procédé de coloration ou de maquillage de la peau ou des ongles mettant en oeuvre une composition contenant au moins un pigment répondant à la formule telle que définie ci-dessus.

Les exemples suivants sont destinés à illustrer l'invention sans pour autant présenter un caractère limitatif.

**Exemple**

Préparation 5H-dinaphto[2,3-a:2',3'-c]carbazole-6,11,12,17-tétrone

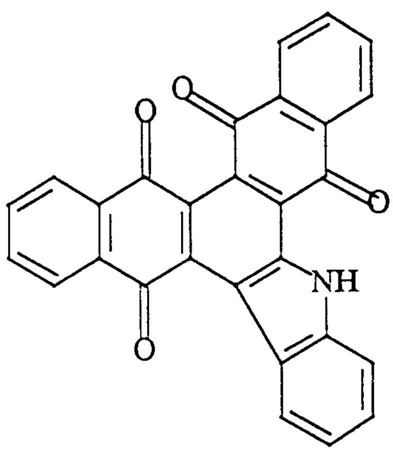

Dans un réacteur de 2 litres muni d'une agitation mécanique, d'un thermomètre et d'un réfrigérant, on place 100 g de 1,4-naphtoquinone (632,3 mmol) en suspension dans 1000 ml d'acide acétique. On ajoute 92,9 g (1,25 éq) d'indole et on chauffe sous agitation à 92°C pendant 8 heures. Après une nuit à température ambiante, on filtre le milieu réactionnel sur verte fritté. On lave le précipité à l'éthanol et à l'acétone. On sèche sous vide. On obtient 35,8 g d'un produit rouge, soit un rendement final de 27%

**Analyses** :

| Analyse élémentaire pour $C_{28}H_{13}NO_4$ | | | | |
|---|---|---|---|---|
| | %C | H | N | O |
| théorie | 78,61 | 3,04 | 3,27 | 14,97 |
| trouvé | 78,46 | 3,06 | 3,12 | 15,14 |

Point de fusion : > 350°C

Les spectres RMN sont enregistrés sur un BRUKER WM 250

$^1$H RMN (DMSO-$d_6$), δ(ppm) : 12,92(1H,s,NH, échangeable Dt) 8,32 (1H,d,H-4"), 8,09 à 8,30 (4H,m,H-5,H-5' H-8, H-8')7,94 à 8,03 (5H,m,H-6,H-6',H-7, H-7',H-7") 7,69 (1H,t,H-6") 7,42 (1H,t,H-5")

Les spectres de masse sont enregistrées sur un SM, m/z : 427 ($M^{+\cdot}$), 399, 371, 342, 314

| ANALYSES ou TESTS | RESULTATS |
|---|---|
| S.M EI-DI | Ion moléculaire et Pic de base m/z = 427 $M^{+\cdot}$ |
| Analyse élémentaire | Théorie % C 78,61 H 3,04 N 3,27 O14,97 |
| | Trouvé % 78,56 3,22 3,18 14,94 |
| Mesure de la couleur L a b (t = 0') | L : 47,99 a : 35,16 b : 23,36 |
| GRANULOMETRIE | en Nombre → Moyenne= 2,34µm (écart type 1,04) |
| | en Volume → Moyenne= 4,37µm (écart type 2,53) |
| SOLUBILITE | Insoluble dans l'eau et dans la plupart des solvants organiques |
| STABILITE pH | pH = 5 et pH = 9 BONNE |

Les exemples suivants sont destinés à illustrer les formulations cosmétiques.

**Exemples d'application**

**Exemples de rouges à lèvres**

| | EXEMPLE 1 TEINTE ROUGE | EXEMPLE 2 TEINTE ROSE |
|---|---|---|
| - Huile de ricin | qsp 100 | qsp 100 |
| - (Butyl Hydroxy Toluène) | 0,16 | 0,16 |
| - Lanoline liquide | 17,5 | 17,5 |
| - Cire microcristalline | 15 | 15 |
| - Triglycérides d'acide gras (capryliques / capriques) | 11 | 11 |
| - Béhénate d'octyl glycéryl | 11 | 11 |
| - Pigment de l'exemple de préparation 1 | 10 | 5 |
| - Oxyde de titane | - | 5 |

Le(s) pigment(s) est (sont) dispersé(s) dans la phase grasse fondue

Le maquillage obtenu est transparent et brillant.

## Revendications

1. Utilisation à titre de pigment dans des compositions cosmétiques du 5H-dinaphto[2,3-a:2',3'-c]carbazole-6,11,12,17-tétrone.

2. Utilisation selon la revendication 1, caractérisée par le fait que les compositions cosmétiques sont constituées par des compositions de fonds de teint, de crèmes teintées, des mascaras, des fards à joues ou à paupières, des rouges à lèvres et des vernis à ongles.

3. Composition cosmétique destinée au traitement cosmétique des matières kératiniques, caractérisée par le fait qu'elle contient dans un milieu cosmétiquement acceptable au moins un pigment constitué par le 5H-dinaphto[2,3-a:2',3'-c]carbazole-6,11,12,17-tétrone de formule (I)

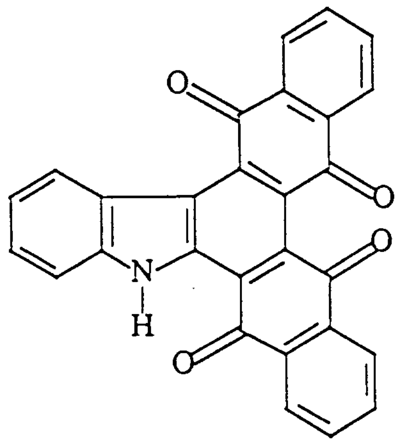

(I)

4. Composition selon la revendication 3, caractérisée par le fait que le pigment de formule (I) est présent en une concentration comprise entre 0,1 et 80% et de préférence entre 1 et 30% en poids par rapport au poids total de la composition.

5. Composition selon l'une quelconque des revendications 3 et 4, caractérisée par le fait que la composition se présente sous forme de lotion, de lotion épaissie, de gel, de crème, de lait, de poudre de stick éventuellement conditionnée en aérosol sous forme de spray ou de mousse.

6. Composition selon l'une quelconque des revendications 3 à 5, caractérisée par le fait qu'elle se présente sous forme d'une émulsion huile-dans-eau ou eau-dans-huile, de dispersion liposomale ou de préparation solide.

7. Composition selon l'une quelconque des revendications 3 à 6, caractérisée par le fait qu'elle contient des corps gras, des solvants organiques, des silicones, des épaississants, des adoucissants, des tensio-actifs, des filtres solaires, des agents anti-mousses, des agents hydratants, des parfums, des conservateurs, des agents anti-oxydants, des charges, des séquestrants, des agents de traitement, des propulseurs, des agents alcalinisants ou acidifiants ou d'autres pigments.

8. Composition selon l'une quelconque des revendications 3 à 6, caractérisée par le fait qu'elle contient des nanopigments d'oxydes métalliques.

9. Composition selon la revendication 3, caractérisée par le fait qu'elle est destinée à être utilisée pour la coloration des ongles et qu'elle se présente sous forme d'une dispersion du pigment dans un solvant cosmétiquement acceptable contenant une ou plusieurs résines.

10. Procédé de maquillage de la peau caractérisé par le fait que l'on applique sur la peau au moins une composition telle que définie à l'une quelconque des revendications 3 à 8 et contenant à titre de pigment au moins du 5H-dinaphto[2,3-a:2',3'-c]carbazole-6,11,12,17-tétrone.

11. Procédé de coloration des ongles caractérisé par le fait que l'on applique sur les ongles au moins une composition contenant dans un solvant cosmétiquement acceptable, une ou plusieurs résines et au moins un pigment constitué par le 5H-dinaphto[2,3-a:2',3'-c]carbazole-6,11,12,17-tétrone.

## Claims

1. Use as a pigment, in cosmetic compositions, of 5H-dinaphtho[2,3-a:2',3'-c]carbazole-6,11,12,17-tetrone.

2. Use according to Claim 1, characterized in that the cosmetic compositions consist of foundation compositions, tinted creams, mascaras, blushers or eyeshadows, lipsticks and nail varnishes.

3. Cosmetic composition intended for the cosmetic treatment of keratinous matter, characterized in that it contains, in a cosmetically acceptable medium, at least one pigment consisting of 5H-dinaphtho[2,3-a:2',3'-c]carbazole-6,11,12,17-tetrone of formula (I)

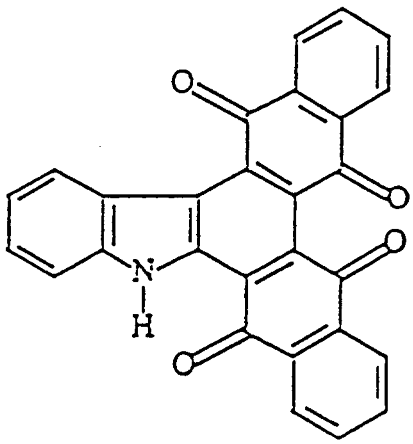

(I)

4. Composition according to Claim 3, characterized in that the pigment of formula (I) is present at a concentration of between 0.1 and 80%, and preferably between 1 and 30%, by weight relative to the total weight of the composition.

5. Composition according to either of Claims 3 and 4, characterized in that the composition is in the form of a lotion, a thickened lotion, a gel, a cream, a milk, a powder or a stick, or is optionally packaged as an aerosol in the form of a spray or a foam.

6. Composition according to any one of Claims 3 to 5, characterized in that it is in the form of an oil-in-water or water-in-oil emulsion, a liposomal dispersion or a solid preparation.

7. Composition according to any one of Claims 3 to 6, characterized in that it contains fatty substances, organic solvents, silicones, thickeners, emollients, surfactants, sunscreens, anti-foaming agents, moisturizing agents, fragrances, preserving agents, antioxidants, fillers, sequestering agents, treatment agents, propellants, acidifying or basifying agents, or other pigments.

8. Composition according to any one of Claims 3 to 6, characterized in that it contains metal oxide nano-pigments.

9. Composition according to Claim 3, characterized in that it is intended to be used for colouring the nails and in that it is in the form of a dispersion of the pigment in a cosmetically acceptable solvent containing one or more resins.

10. Process for making up the skin, characterized in that at least one composition as defined in any one of Claims 3 to 8 and containing, as pigment, at least 5H-dinaphtho[2,3-a:2',3'-c]carbazole-6,11,12,17-tetrone is applied to the skin.

11. Process for colouring the nails, characterized in that at least one composition containing, in a cosmetically acceptable solvent, one or more resins and at least one pigment consisting of 5H-dinaphtho[2,3-a:2',3'-c]carbazole-6,11,12,17-tetrone is applied to the nails.

**Patentansprüche**

1. Verwendung von 5H-Dinaphtho(2,3-a:2',3'-c)carbazol-6,11,12,17-tetraon als Pigment in kosmetischen Zusammensetzungen.

2. Verwendung gemäß Anspruch 1,
dadurch **gekennzeichnet**, daß
die kosmetischen Zusammensetzungen Zusammensetzungen für Teint-Schminkgrundlagen, gefärbte Creme-Produkte, Wimperntuschen, Schminkprodukte für Wangen oder Augenlider, Lippenstifte und Nagellacke darstellen.

3. Kosmetische Zusammensetzung zur kosmetischen Behandlung keratinischer Materien,
dadurch **gekennzeichnet**, daß
sie in einem kosmetisch geeigneten Milieu mindestens ein Pigment aus 5H-Dinaphtho(2,3-a:2',3'-c)carbazol-6,11,12,17-tetraon der Formel (I) enthält:

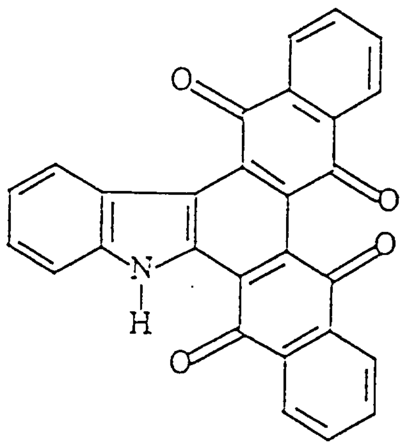

(I)

4. Zusammensetzung gemäß Anspruch 3,
dadurch **gekennzeichnet**, daß
das Pigment der Formel (I) in einer Konzentration von 0,1 bis 80 und vorzugsweise von 1 bis 30 Gew.% vorhanden ist, bezogen auf das Gesamtgewicht der Zusammensetzung.

5. Zusammensetzung gemäß jedem der Ansprüche 3 und 4,
dadurch **gekennzeichnet**, daß
sie in Form einer Lotion, verdickten Lotion, eines Gels, einer Creme, Milch, eines Puder-Stifts vorliegt oder gegebenenfalls als Aerosol in Form eines Spray oder Schaums zubereitet ist.

6. Zusammensetzung gemäß jedem der Ansprüche 3 bis 5,
dadurch **gekennzeichnet**, daß
sie in Form einer Öl-in-Wasser- oder Wasser-in-Öl-Emulsion, liposomalen Dispersion oder einer Feststoff-Zubereitung vorliegt.

7. Zusammensetzung gemäß jedem der Ansprüche 3 bis 6,
dadurch **gekennzeichnet**, daß
sie Fettkörper, organische Lösungsmittel, Silikone, Verdickungsmittel, Weichmacher, oberflächenaktive Mittel, Filterstoffe für Sonnenstrahlen, Antischaummittel, hydratisierende Mittel, Parfümstoffe, Konservierungsstoffe, Antioxidantien, Beaufschlagungsmittel, Sequestriermittel, Behandlungsmittel, Treibmittel, alkalisch oder sauer stellende Mittel oder weitere Pigmente enthält.

8. Zusammensetzung gemäß jedem der Ansprüche 3 bis 6,
dadurch **gekennzeichnet**, daß
sie Nanopigmente metallischer Oxide enthält.

**9.** Zusammensetzung gemäß Anspruch 3, dadurch **gekennzeichnet**, daß sie dazu bestimmt ist, zur Färbung von Nägeln verwendet zu werden, und in Form einer Dispersion des Pigments in einem kosmetisch geeigneten Lösungsmittel vorliegt, das eines oder mehrere Harze enthält.

**10.** Verfahren zum Schminken der Haut, dadurch **gekennzeichnet**, daß man auf die Haut mindestens eine in jedem der Ansprüche 3 bis 8 definierte Zusammensetzung aufbringt, die als Pigment mindestens 5H-Dinaphtho(2,3-a:2',3'-c)carbazol-6,11,12,17-tetraon enthält.

**11.** Verfahren zur Färbung der Nägel, dadurch **gekennzeichnet**, daß man auf die Nägel mindestens eine Zusammensetzung aufträgt, die in einem kosmetisch geeigneten Lösungsmittel ein oder mehrere Harze und mindestens ein Pigment aus 5H-Dinaphtho(2,3-a:2',3'-c)carbazol-6,11,12,17-tetraon enthält.